Europäisches Patentamt

**European Patent Office**

**Office européen des brevets**

(11) Publication number: **0 107 440**
**A1**

## EUROPEAN PATENT APPLICATION

(21) Application number: **83306157.5**

(22) Date of filing: **12.10.83**

(51) Int. Cl.³: **A 61 M 5/14, F 04 B 43/12**

(30) Priority: **15.10.82 US 434646**

(71) Applicant: **BAXTER TRAVENOL LABORATORIES, INC., One Baxter Parkway, Deerfield, IL 60015 (US)**

(43) Date of publication of application: **02.05.84 Bulletin 84/18**

(72) Inventor: **Sukel, Gerald J., 909 Abbington Drive, Crystal Lake Illinois 60014 (US)**

(74) Representative: **MacGregor, Gordon et al, ERIC POTTER & CLARKSON 14 Oxford Street, Nottingham, NG1 5BP (GB)**

(84) Designated Contracting States: **BE CH DE GB IT LI**

(54) **Roller pump set.**

㊼ A roller pump set is provided for insertion into a roller pump housing (36). The roller pump set includes a tubing bridge (20) with a fixed loop segment (26) of silicone tubing connected to one side (28) of the bridge for looping around the rollers (34) and inlet (12, 16) and outlet tubing (22) connected to the other side (18) of the bridge and communicating with the fixed loop segment. The roller pump housing carries indexing means (50) which cooperate with polarizing means (70) on the tubing bridge. In this manner, incorrect pump set insertion in the roller pump housing is prevented.

-1-

ROLLER PUMP SET

This invention concerns a roller pump set for use in roller pump apparatus and is particularly concerned with such apparatus for use in a medical environment.

## BACKGROUND ART

Enteral feeding is a medical procedure for patients who do not require intravenous feeding, but cannot take food or liquid in the normal manner. Typically, an enteral feeding container has a drip chamber and tubing which is connected to an enteral feeding catheter intended to communicate to the patient's stomach through the patient's nasal passage. Often the tubing is coupled to a roller pump, which serves to pump the liquid in the enteral feeding container through the drip chamber and tubing to the patient. When a roller pump is used to pump the liquid to the patient, it is mandatory that the pump be operated to direct the liquid in the right direction. If the pumping is reversed, the patient may be aspirated with serious consequences.

Although the above example relates to enteral roller pumps, it is to be understood that the invention is applicable to infusion pump systems also.

It is an object of the present invention to provide a roller pump set which is constructed so it is prevented from being incorrectly coupled to a roller pump.

Another object of the present invention is to provide a roller pump set that is constructed to be easy for the operator to couple it with a roller pump.

A further object of the present invention is to provide a roller pump system including a roller pump and roller pump set which cooperate to prevent incorrect insertion of the roller pump set with respect to the roller pump.

A still further object of the present invention is to provide a roller pump set which is relatively simple in construction and easy to manufacture.

Other objects and advantages of the present invention will become apparent as the description proceeds.

## DISCLOSURE OF THE INVENTION

In accordance with the present invention, a roller pump system is provided including a roller pump carried on a roller pump housing, and a pump set for engagement with the roller pump. The pump set comprises a tubing bridge, a fixed loop segment of tubing for looping around the roller, and inlet and outlet tubing communicating with the fixed loop segment. The fixed loop segment and the inlet and outlet tubing are connected to the tubing bridge. The housing has indexing means and the bridge has polarizing means. The polarizing means are cooperative with the indexing means to prevent incorrect pump set insertion in the roller pump housing.

In the illustrative embodiment, the inlet tubing comprises a drip chamber and the inlet and outlet tubing is connected to the top side of the bridge. The fixed loop segment is connected to the bottom side of the bridge.

In the illustrative embodiment, the indexing means and the polarizing means comprise a complementary protuberance and receptacle. The protuberance comprises at least one pin carried by the housing and the receptacle comprises a member carried by the bridge and defining a slot for receiving the pin when the pump set is inserted properly.

In the illustrative embodiment, the bridge is formed as a one-piece molded plastic unit and the fixed loop segment is formed of silicone tubing. The inlet tubing comprises a drip chamber connected to the bridge and a length of tubing extending from the inlet of the drip chamber with an enteral feeding container connected to the inlet end of the length of tubing.

A more detailed explanation of the invention is provided in the following description and claims, and is illustrated in the accompanying drawings.

BRIEF DESCRIPTION OF THE DRAWINGS

Figure 1 is a front view of a roller pump system constructed in accordance with the principles of the present invention.

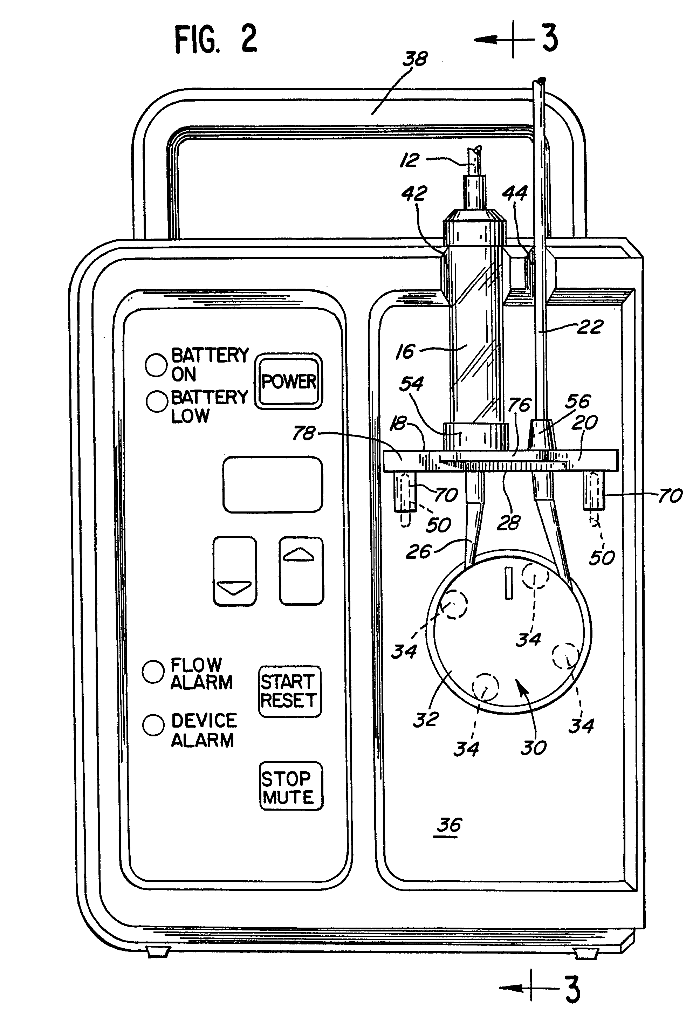

Figure 2 is a front view of the roller pump housing of Figure 1.

Figure 3 is a cross-sectional view thereof, taken along the plane of the line 3-3 of Figure 2.

Figure 4 is a rear view of the bridge used with the roller pump system of Figure 1.

Figure 5 is a cross-sectional view thereof, taken along the plane of the line 5-5 of Figure 4.

Figure 6 is a top view thereof.

Figure 7 is a broken perspective view thereof.

DESCRIPTION OF SPECIFIC EMBODIMENT

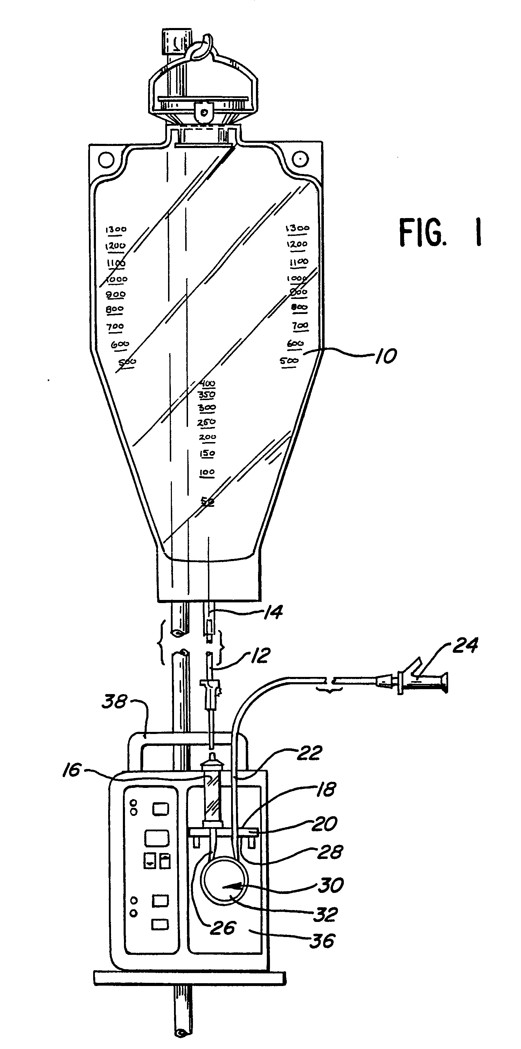

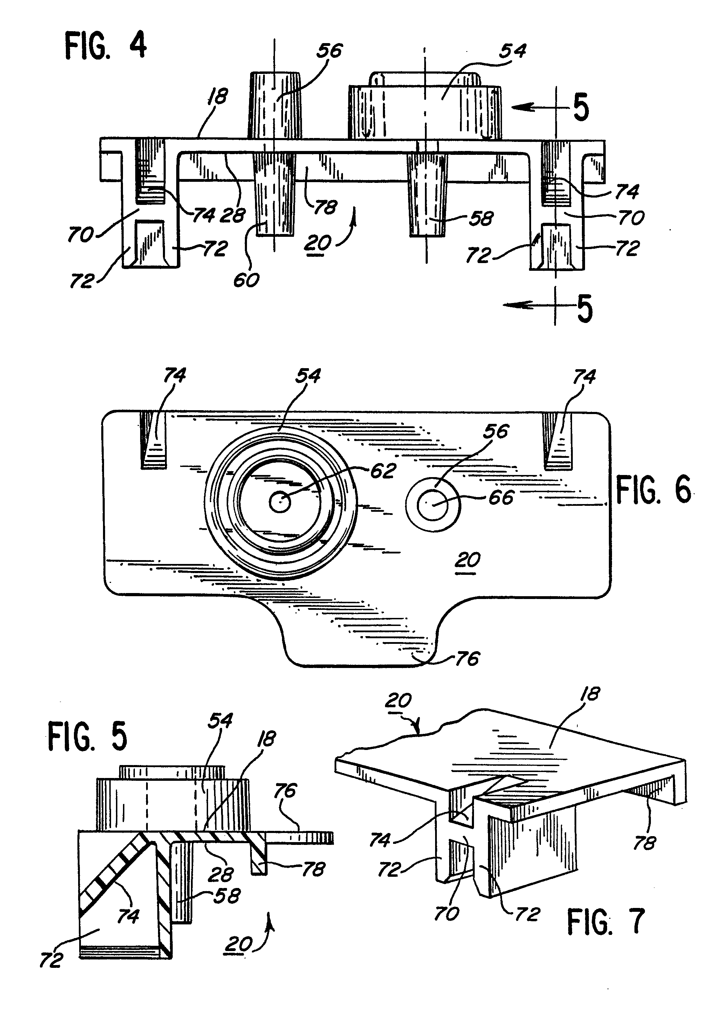

Referring to Figure 1 in particular, a roller pump system is shown therein including an enteral feeding container 10, tubing 12 from outlet port 14 with tubing 12 including a drip chamber 16 connected to the top side 18 of a bridge 20. Bridge 20 is formed as a one-piece molded plastic unit and will be described in more detail below in connection with Figures 4-7. Also connected to top side 18 of bridge 20, in spaced relation to drip chamber 16, is outlet tubing 22 having an enteral catheter 24 at its distal end.

A fixed loop segment 26, formed of silicone tubing, is connected to the bottom 28 of bridge 20. Fixed loop segment 26 communicates with drip chamber 16 and outlet tubing 22 as illustrated and the fixed loop is adapted for coupling to roller pump 30 which comprises a rotor 32 carrying four rollers 34.

Rotor 32 is carried on a pump housing 36 which carries appropriate switches and condition indicia, and is adapted for resting upon a shelf in the upright position as illustrated. Housing 36 has a carrying handle 38 and a rear chamber 40 in which the motor 41 for driving rotor 32 is located. Housing 36 defines slots 42 and 44 into which drip chamber 16 and outlet tubing 22, respectively, are held with a pressure fit.

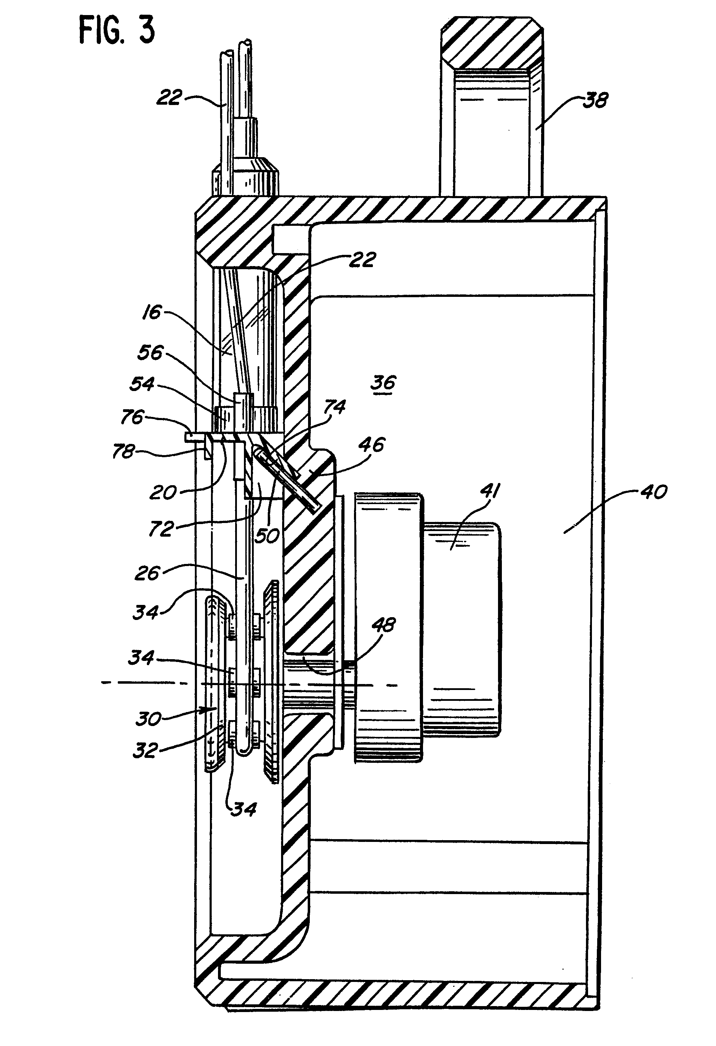

Housing 36 has a supporting wall 46 which defines an opening 48 for the motor shaft which drives rotor 32. A pair of pins 50 are fastened into supporting wall 46 and the pins 50 extend upwardly at a 45° angle as illustrated in Figure 3, to support bridge 20. Pins 50 serve as polarizing means to permit indexing of bridge 20 so that it can be seated in the pump housing in only one possible position. Referring now to Figures 4-7, it can be seen that bridge 20 carries a fitting 54 for receiving drip chamber 16, a fitting 56 for receiving outlet tubing 22, a fitting 58 for receiving one end of silicone tubing 26 and a fitting 60 for receiving the other end of silicone tubing 26. Fittings 54 and 58 communicate with each other via opening 62 (Figure 6) while fittings 56 and 60 communicate with each other via opening 66.

Bridge 20 also has indexing means in the form of a pair of downwardly extending members 70. Each of these members is spaced from the other substantially the entire width of the bridge and is adapted for receiving one of the pins 50. Each of downwardly extending members 70 comprises a pair of vertical parallel walls 72 and a bridging angled portion 74 which extends upwardly in the forward direction as illustrated in Figure 7. The front of bridge 20 has an outwardly extending lip 76 and also a downwardly extending flange 78.

In one form of the invention, the operator may be provided the roller pump set as a complete unit, including the connected enteral feeding container, inlet tubing (including drip chamber), bridge, fixed loop segment, outlet tubing and enteral catheter. The set would be inserted into housing 36 by looping the fixed loop segment 26 around the rollers 34 of roller pump 30 and placing the bridge 20 against wall 46 so that pins 50 enter the slotted receptacle formed by walls 72 and angled portion 74, with the underside of angled portions 74 resting upon the pins 50. Drip chamber 16 and outlet tubing 22 are then placed into slots 42 and 44, respectively, and the pump is ready for operation.

It can be seen that in view of the receptacles 70 carried by the bridge and the pins 50 carried by the housing, the set is prevented from being coupled to the pump in the wrong direction. Also, lip 76 aids in preventing the set from being coupled to the pump in the wrong direction, because bridge 20 cannot be set in place with lip 76 facing wall 46. Furthermore, it can be seen that the set is easy for an operator to place into the roller pump housing in cooperation with the roller pump.

Although an illustrative embodiment of the invention has been shown and described, it is to be understood that various modifications and substitutions may be made by those skilled in the art without departing from the novel spirit and scope of the present invention.

CLAIMS

1.    A roller pump set for insertion into a roller pump housing (36) carrying a roller pump and indexing means (50), the roller pump set comprising a tubing bridge (20), a fixed loop segment (26) of tubing, for looping around the rollers (34) of the roller pump, inlet (12, 16) and outlet (22) tubing communicating with the fixed loop segment; the fixed loop segment and the inlet and outlet tubing being connected to the tubing bridge; said bridge having polarizing means (70), and said polarizing means being arranged to be cooperative with said indexing means to prevent incorrect pump set insertion in the roller pump housing.

2.    A roller pump set according to Claim 1, wherein the inlet tubing comprises a drip chamber (16).

3.    A roller pump set according to Claim 1 or 2, wherein the bridge has a bottom side (28) and a top side (18), and the fixed loop segment (26) is connected to the bottom side and the inlet and outlet tubing is connected to the top side.

4.    A roller pump set according to Claim 1, 2 or 3, wherein said inlet tubing comprises a drip; chamber (16) connected to the bridge (20), a length of tubing (12) extending from the inlet of the drip chamber and an enteral feeding container (10) connected to the inlet end of the length of tubing.

5.    A roller pump set according to any preceding claim, wherein said bridge (20) is formed as a one-piece molded plastics unit and said fixed loop segment (26) is formed of silicone tubing.

6.    A roller pump apparatus comprising a housing (36) carrying a roller pump (34) and indexing means (50) and also comprising a roller pump set according to any preceding claim, wherein said indexing means (50) and said polarizing means (70) comprise a complementary

0107440

-8-

protuberance (50) and receptacle (70).

7.   A roller pump apparatus according to Claim 6, said protuberance comprising at least one pin (50) carried by said housing and said receptacle comprising a member (70) carried by said bridge (20) and defining a slot (72, 74) for receiving said pin when the pump set is inserted properly.

0107440

FIG. 1

1300  1300
1200  1200
1100  1100
1000  1000
900   900
800   800
700   700
600   600
500   500

400
350
300
250
200
150
100
50

10

14
12
24
38
16
22
18
20
28
30
32
26
36

FIG. 2

0107440

2/4

0107440

FIG. 3

0107440

FIG. 4

FIG. 6

FIG. 5

FIG. 7

0107440

## European Patent Office

**EUROPEAN SEARCH REPORT**

Application number

EP 83 30 6157

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-2 012 373 (MEDICAL SCIENCES INTERNATIONAL N.V.) | | A 61 M 5/14<br>F 04 B 43/12 |
| | --- | | |
| A | FR-A-2 459 663 (LIGATURES PETERS) | | |
| | --- | | |
| A | FR-A-2 316 509 (MALBEC) | | |
| | ----- | | |

**TECHNICAL FIELDS SEARCHED (Int. Cl. ³)**

A 61 M
F 04 B

The present search report has been drawn up for all claims

| Place of search | Date of completion of the search | Examiner |
|---|---|---|
| THE HAGUE | 10-01-1984 | KNAUER F.E. |